# EUROPEAN PATENT SPECIFICATION

(11) **EP 1 492 471 B1**
(45) Date of publication and mention of the grant of the patent: **10.12.2008**
(21) Application number: 03718287.0
(22) Date of filing: 09.04.2003
(51) Int. Cl.: A61F 2/00, A61F 2/06

(54) **HYBRID STENT**
MISCH-STENT
STENT HYBRIDE

(30) Priority: 10.04.2002 US 63315
(43) Date of publication of application: 05.01.2005
(73) Proprietor: Boston Scientific Limited, St. Michael, Barbados, West Indies (BB)
(72) Inventor: BERENSTEIN, Alejandro, New York, NY 10128 (US); EDER, Joseph, C., Los Altos Hills, CA 94024 (US)
(74) Representative: Hauck Patent- und Rechtsanwälte
(86) International application number: PCT/US2003/010876
(87) International publication number: WO 2003/086237

(56) References cited:
- WO-A-00/41633
- WO-A-98/07385
- WO-A-20/04058100
- US-A- 5 061 275

## Description

### Background of Invention

The use of stents to maintain the patency of bodily lumens is well known. Stents are typically delivered via a catheter in an unexpanded configuration to a desired bodily location. Once at the desired bodily location, the stent is expanded and implanted in the bodily lumen. The stent may self-expand or may be mechanically expanded. Where a self-expanding stent is used, the stent is typically retained on the catheter via a retention device such as a sheath. The stent may be deployed by retracting the sheath from over the stent. Where a mechanically expandable stent is used, a radially outward force is typically applied to the stent to expand it. The force may be applied via an expandable member such as a balloon or via any other mechanical device.

Stents are used in an array of bodily vessels including the coronary arteries, the peripheral arteries, arteries of the neck, cerebral arteries, veins, biliary ducts, urethras, ureters, fallopian tubes, bronchial tubes, the trachea, the esophagus, the prostate and bowels or any other tubular organs.

Currently available stents include tubular stents such as the NIR™stent as well as coil stents. Coil stents typically are formed of a wire or strand which has been wound into a coil shape. Coil stents typically have a small surface area and can exhibit a high degree of flexibility, including bendability and longitudinal flexibility which facilitates delivery of the stent through tortuous bodily vessels or tubular structures.

The use of coil stents is particularly appealing for use in containing embolic materials within aneurysms without occluding perforating vessels. In the past, aneurysms of peripheral arteries and arteries of the neck have been treated with open walled stents. Open walled stents are believed to slow the blood flow in the aneurismal sac leading to the formation of clots and fibrous masses which occlude the aneurysm.

Typically, however, coil stents are not expandable. The post deployment diameter of the coil stent is typically the same as the diameter of the coil stent prior to being loaded onto a delivery catheter. As such, coil stents must be very closely matched in size to the diameter of the vessel in which they will be deployed. If the size of the coil stent is not properly matched to the vessel, the stent may not be able to properly anchor in the vessel.
Document WO 00/41633 A1 (corrected version) discloses a device for forming a vascular anasthomosis. In one embodiment the stent comprises a stent body having two frame elements at one end. The frame elements are made of open rings connected by a band extending in the longitudinal direction of the frame elements.
The problem of the invention is to provide coil stents which are flexible and which can be easily anchored within a vessel.
The problem is solved according to claim 1.
Without limiting the scope of the invention a brief summary of the claimed embodiments of the invention is set forth below. Additional details of the summarized embodiments of the invention and/or additional embodiments of the invention may be found in the Detailed Description of the Invention below.

### Summary of Invention

In one embodiment, the invention is directed to a stent comprising a plurality of segments, including at least one segment which is in the form of a coil and at least one segment which is in a form other than a coil and which is balloon expandable and/or self-expandable.
The stent may be provided in a variety of embodiments. In one embodiment, the stent has a first end segment and a second end segment. Each of the first and second end segments is in a form other than a coil and is balloon expandable or self-expandable. The stent may comprise only one segment which is in the form of a coil and which connects the first and second end segments. The first and second end segments may be self-expandable or balloon expandable. Where self-expanding segments are used, the self-expanding segments may be made of shape memory materials in order to self-expand or may be made of braided filaments which self-expand. Where balloon expandable segments are used, desirably, the first and second end segments are each in the form of a tube comprising a plurality of interconnected serpentine segments.
The invention is also directed to a stent comprising a coil segment and a tubular, non-coil segment. In some embodiments, the non-coil coil segment will be balloon expandable. In other embodiments, the tubular, non-coil segment will be self-expandable. Typically, both the first end and the second end of the stent will be a tubular, non-coil segment.
Typically, in the various embodiments of the invention, the segment which is in the form of a coil will be made of spring steel. Other suitable materials including platinum and stainless steel coated with platinum may also be used.
The invention is also directed to a method of manufacturing a stent comprising the steps of providing a coil segment and a non-coil segment and attaching the coil segment to the non-coil segment. Desirably, the coil segment will be adhesively bonded to the non-coil segment or welded thereto.
Additional details and/or embodiments of the invention are discussed below.

### Brief Description of Drawings

Figure 1a shows a schematic illustration of an inventive stent.
Figure 1b shows a schematic illustration of an inventive stent.
Figure 2 shows a schematic illustration of an inventive stent.
Figure 3 is a perspective view of a stent segment which may be used in an inventive stent.
Figure 4 is a side view of a stent segment which may be used in an inventive stent.
Figure 5 is a side view of another stent segment which may be used in an inventive stent.
Figure 6a is a perspective view of a commercially available stent segment which may be used in an inventive stent.
Figure 6b is a perspective view of an inventive stent.
Figure 7a is a side view of a coil segment for use in an inventive stent.
Figure 7b shows an enlarged view of portion 7b of the coil segment of Fig. 7a.
Figure 8 is a side view of a coil segment of an inventive stent in accordance with the invention.
Figure 9 is a side view of a coil segment of a vena cava filter in accordance with the invention.

Figure 10 is a side view of a catheter with an inventive stent disposed thereabout with parts cut away.

Figure 11 is a side view of an inventive stent disposed about a balloon catheter in a bodily vessel.

Figure 12 is a side view of an inventive stent seated in a vessel.

Figure 13a is a schematic view of an inventive bifurcated stent.

Figure 13b is a schematic view of an inventive bifurcated stent.

Figures 14a and 14b are schematic illustrations showing an inventive bifurcated stent pre and post deployment.

Figure 14c is a schematic illustration of showing another inventive bifurcated stent.

Figure 15 is a side view of an inventive stent seated in a vessel in the region of an aneurysm.

Figure 16 is a side view with parts cut away of an inventive stent such as that shown in Fig. 1a with a covering over the entirety of the stent.

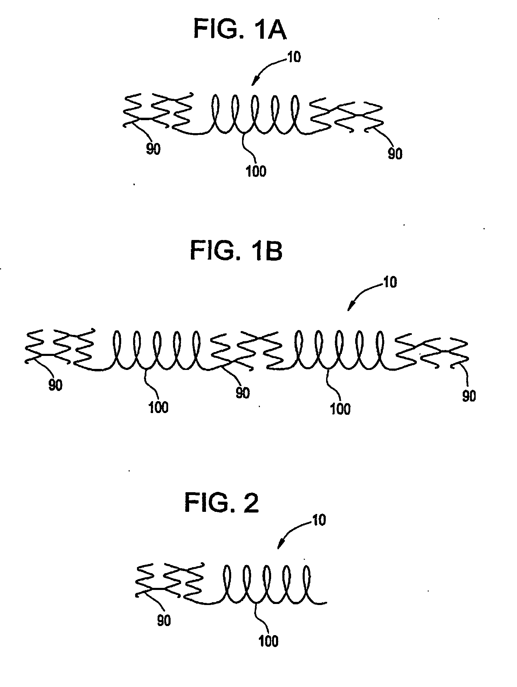

Figure 1 7 is a side view with parts cut away of an inventive stent such as that shown in Fig. 1a with a portion of the stent having a covering.

### Detailed Description

While this invention may be embodied in many different forms, there are described in detail herein specific preferred embodiments of the invention. This description is an exemplification of the principles of the invention and is not intended to limit the invention to the particular embodiments illustrated.

For the purposes of this disclosure, like reference numerals in the figures shall refer to like features unless otherwise indicated. Also for the purposes of this disclosure, the term "non-coil segment" shall be understood to mean a stent segment which is expandable mechanically, such as by balloon, self-expandable or otherwise expandable. Also, the term "coil segment" excludes segments which are in the form of a multiplicity of wires or strands which are woven or braided such as that disclosed in US 5,061,275. Even if not clearly visible*,* the end segments are always formed of a plurality of braided filaments.

In one embodiment, the invention is directed to a stent such as that shown generally at 10 in Fig. 1a, comprising a plurality of segments, including at least one segment 100 which is in the form of a coil and at least one segment 90 which is in a form other than a coil and which is balloon expandable or self-expandable. In the stent of Fig. 1a, two balloon expandable or self-expanding segments 90 are provided, one at each end of the stent. Other arrangements of the coil segment and the non-coil segment are also within the scope of the invention.

In embodiment shown in Fig. 1b, inventive stent 10 includes two coil segments 100 and three non-coil segments 90. Each coil segment is disposed between two non-coil segments. Longer stents with alternating coil and non-coil segments are within the scope of the invention as well. By way of a non-limiting example, an inventive stent may be provided having four, five, six or more coil segments which alternate with non-coil segments. The non-coil segments may be balloon expandable and/or self-expanding. For example, all of the non-coil segments 90 shown in Fig. 1b may be self-expanding or all of the non-coil segments may be balloon expandable or some of the non-coil segments may be balloon expandable and some self-expanding. As an example of the latter, the end non-coil segments may be self-expanding and the middle non-coil segment may be balloon expandable. As another example of the latter, the end non-coil segments may be balloon expandable and the middle non-coil segment may be self-expanding.

The inventive stents, more generally, may have at least N segments which are balloon expandable or self-expanding and M coil segments where N and M are integers greater than or equal to one and N and M desirably equal one another or desirably differ from one another by 1. The N segments may consist entirely of balloon expandable segments, entirely of self-expanding segments or may consist of a combination of balloon expandable segments and self-expanding segments. In one non-limiting example, an inventive stent has two self-expanding segments, one at each end of the stent, one balloon expandable section disposed between the two self-expanding segments and two coil segments, each coil segment disposed between adjacent balloon expandable and/or self-expanding segments. In another non-limiting example, an inventive stent has two self-expanding segments, one at each end of the stent, one balloon expandable section disposed between the two self-expanding segments and four coil segments, each balloon expandable and/or self-expanding segment disposed between adjacent coil segments. The inventive stent may also be provided with balloon expandable segments at the ends and a self-expanding segment in the middle.

As yet another example, as shown in Fig. 2, the invention is also directed to stents having only a single balloon expandable or self-expanding segment 90 and a single coil segment 100. As yet another example, not shown, an inventive stent having three or more balloon expandable and/or self-expanding segments and three or more coil segments may be provided.

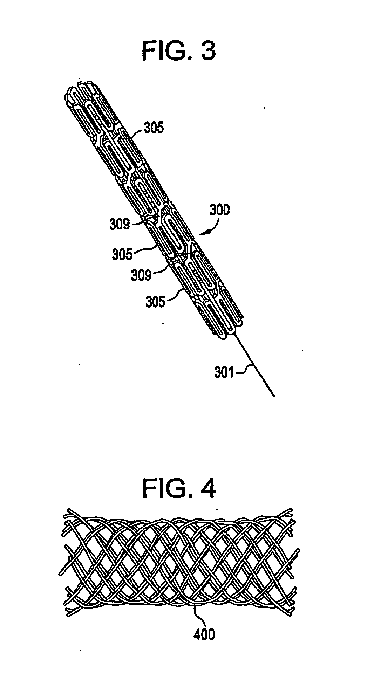

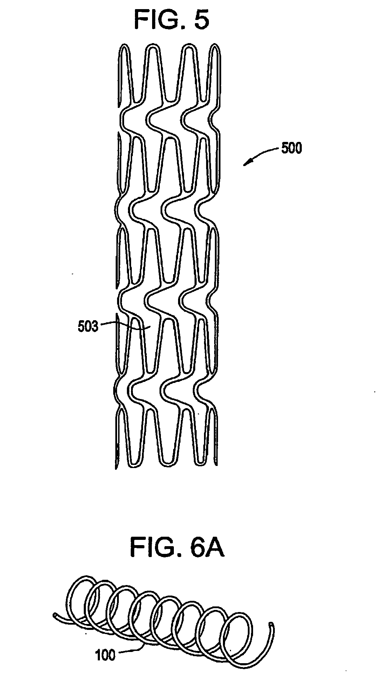

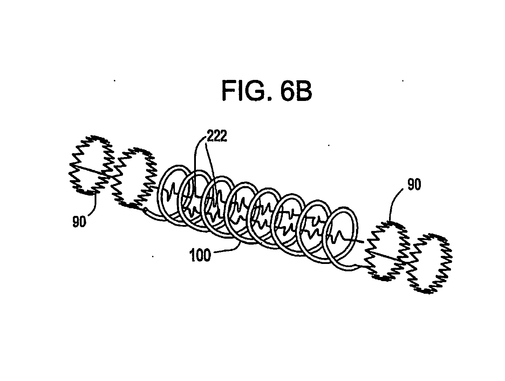

As discussed above, the first and second end segments, and more generally, the non-coil segments, may be self-expandable or balloon expandable. Where self-expanding segments are used, the self-expanding segments may be made of shape memory materials in order to self-expand or may be made of braided filaments. Suitable shape memory materials include shape memory metals such as nitinol and shape memory polymers. An example of a self-expanding segment which may be used as one of the non-coil segments is disclosed in W09626689 and shown at 300 in Fig. 3. The stent segment of Fig. 3, made of nitinol, includes a plurality of serpentine segments 305 extending about the longitudinal axis 301 of the stent and a plurality of members 309 which extend between adjacent serpentine segments. Tubular segments with other geometries, as are known in the art, may also be used. An example of a braided self-expanding segment which may be used in the practice of the invention is shown at 400 in Fig. 4 and is described in greater detail in US 5,061, 275.
Where balloon expandable segments are used, desirably, the balloon- expandable segments are in the form of a tube comprising a plurality of interconnected serpentine segments. As a non-limiting example, a segment having a configuration such as that shown in Fig. 3 and made of stainless steel may be used. Another non-limiting example is shown generally at 500 in Fig. 5. Segment 500 is in the form of a tube with a plurality of openings 503 therein. Segment 500 may be made of stainless steel or other suitable stent materials including metals such as titanium, tantalum, MP-35N, elgiloy, platinum, platinum-tungsten, platinum-nickel, platinum-rhenium, gold, tantalum and titanium aluminide, polymers such as polyurethane, silicone elastomers, polytetrafluoroethylene and combinations thereof. Tubular segments with other geometries, as are known in the art, may also be used.
Examples of coils which may be used as the coil segment in the inventive stents are described in US 4,553, 545. One such coil is shown at 100 in Fig. 6a. The coil of Fig. 6a may also have adjacent turns of the coil tethered to one another via connector segments 222 as shown in Fig. 6b. Connector segments 222 have one or more bends to provide some slack to allow for expansion of the coil. Connector segments 222 may extend between each of the turns of the coil, as shown in Fig. 6b or between only some of the turns of the coil. The connector segments may extend the entire length of the coil or may extend along only a portion of the coil. In the embodiment of Fig. 6b, two parallel lines of connector segments are provided. Fewer parallel lines of connector segments may be provided and similarly, more parallel lines of connector segments extending between turns may be provided. The connector segments may also be arranged to helically spiral about the coil itself. Coil 100 shown in Fig. 6b form the middle part of a stent with a non-coil portion extending from each end. The connector segments may be welded, adhesively bonded or otherwise connected to the turns of the coil.
The coil segments used in the inventive stents and other medical devices disclosed herein may be made of any suitable metal or polymeric material. An example of a suitable material is spring steel. Other examples of suitable materials include stainless steel, nitinol, platinum, platinum-tungsten, platinum-nickel, platinum-rhenium MP-35N, ELGILOY, gold, tantalum, and titanium and alloys thereof. Suitable polymers include polyurethane, silicone elastomers, polytetrafluoroethylene and combinations thereof. Hydrogels and/or hydrophobic, hydrolytic or biodegradable materials and combinations thereof may also be used. An example of one such material is collagen.

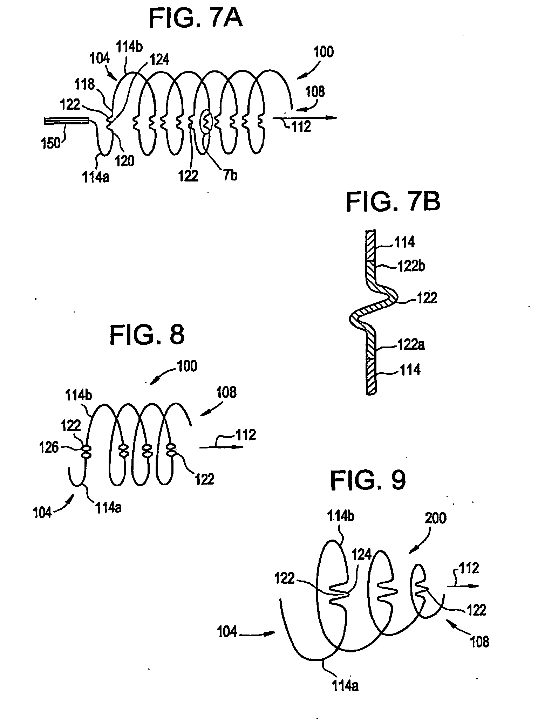

With reference to Fig. 7a, another coil segment such as that shown generally at 100 in Fig. 7a may be used as part of the inventive stents. Coil stent segment 100 is shown in Fig. 7a as it is being deployed from catheter 1 50. Coil stent segment 100 has a proximal end 104, a distal end 108 and a longitudinal axis 112 therethrough. Coil stent segment 100 comprises first curved segment 1 14a and second curved segment 114b. First curved segment 114a and second curved segment 114b arc about longitudinal axis 112 of stent 100. First curved segment 114a and second curved segment 114b have a first end 118 and a second end 120. Coil stent segment 100 further comprises expandable link 122 extending between second end 120 of first curved segment 114a and first end 118 of second curved segment 114b. As shown in Fig.7a, expandable link 122 has a plurality of bends 124 therein. The coil stent segment may be provided in embodiments in which the expandable segment has a single bend and embodiments in which the expandable sections have a serpentine or other bent appearance.

Desirably, as shown in the expanded view of Fig. 7b, the curvature of expandable links 122 at each end 1 22a and 122b of segment 100 is substantially similar to the curvature of the ends of the curved segments 114 to avoid an excess concentration of stress at junctions between the expandable links and the curved segments.

The coil stent segment of Fig. 7a comprises a plurality of expandable links 122. Desirably, nearest neighboring expandable links along the stent are spaced by at least 90 degrees about the longitudinal axis of the stent and more desirably, as shown in Fig. 7a, by at least 180 degrees about the longitudinal axis of the stent.

Coil stent segments comprising a single expandable link may also be used in the inventive stents disclosed herein.

The invention also contemplates other forms for the expandable link of the coil stent segments shown in Figs. 7a and 7b. For example, as shown in Fig. 8, expandable link 122 comprises at least one expandable cell 126 and desirably, a plurality of expandable cells 126. Cells 126 are diamond shaped. Cells of any other suitable, expandable shape may be used as well. For example, the cells may be rectangular or may be defined by a curved shape.

Desirably, as shown in Fig. 8, at least one expandable link is provided per one complete turn of coil stent segment 100 about the longitudinal axis. More desirably, between one and four expandable links are provided per turn of the stent segment. Stated otherwise, nearest neighboring expandable links along stent segment 100 desirably are spaced by between about 90 degrees and 360 degrees apart about the longitudinal axis of the stent segment. In other embodiments of the invention, the coil stent segments may have more than four expandable links per turn or less than one expandable link per turn of the coil stent segment. As an example of the latter, one expandable link may be provided for every two turns of the stent segment about the longitudinal axis of the stent segment.

It is also within the scope of the invention to provide a coil stent segment having at least one expandable link similar to that disclosed in conjunction with Fig. 7a and at least one expandable link similar to that disclosed in conjunction with Fig. 8.

In one embodiment of the invention, the expandable links of the coil segment may be made of stainless steel and the curved segments of the coil segment made of a shape memory material. Suitable shape memory materials include shape memory metals such as nitinol. More generally, the expandable links of the coil segment may be made of a first material and the curved segments of the coil segment made of a second material different from the first material. The expandable links and the curved segments of the coil segment may be joined end-to-end adhesively, via soldering, welding, laser welding, the use of plasma techniques, the use of electron beams or via any other suitable technique. Suitable adhesives include cyanoacrylates and epoxies. Desirably, the curvature of the ends of the expandable links of the coil segment will be substantially similar to the curvature of the ends of the curved segments of the coil segment to avoid an excess concentration of stress at junctions between the expandable links and the curved segments.

The coil segments for use in the inventive stents invention may also be of a form shown in Fig. 7a, comprising a first segment 114a which curves about longitudinal axis 112 of the coil stent segment, a third segment 114b which curves about the longitudinal axis of the coil stent segment and a second segment 122 disposed between first segment 114a and third segment 114b where the first and third segments are formed of a first material and the second segments are formed of a second material different than the first material or differently treated than the first material. The first, second and third segments are joined end-to-end. Desirably, as shown in Fig. 7a, second segment 122 has at least one bend therein. Optionally, second segment 122 may have a plurality of bends therein.

Desirably, the first material is a shape memory material and the second material is stainless steel. The shape memory material may be metal or polymeric. An example of a suitable shape memory material is nitinol. Other suitable metals for use in the inventive stents disclosed herein include L605, MP35N and other metals having a composition of Co 45%-55% by weight, Cr 15%-25% by weight, W 12 %-18.0 % by weight, Ni 8 %-12 % by weight, Fe 1% - 3% by weight and Mn 1% - 2% by weight. L605 has a high modulus of elasticity and is sufficiently radiopaque to allow it to be seen under fluoroscopy. L605 is also MRI (magnetic resonance imaging) compatible. It is noted that L605 may be used in the manufacture of stents of any other known stent designs as well including coil stents and stents comprising a plurality of interconnected bands. L605 may desirably be employed as the second material. The second material may also be a polymeric material. Another suitable second material is nitinol whose superelastic properties have been destroyed.

The first material and second materials used in the coil stent segments may be adhesively joined, joined via soldering, welding, laser welding or any of the other techniques disclosed herein or via any other suitable technique.

The invention is also directed to a medical coil implant, such as that shown at 10 in Fig. 1a, for implantation in a bodily vessel, comprising a coil segment such as that shown at 100 in Fig. 7a and one or more non-coil segments . The coil segment comprises a strand having a plurality of winding segments 114a,b which wind about the longitudinal axis of the implant and a plurality of linking segments 122. Linking segments 122 extend between winding segments 114a,b which are adjacent one another with each linking segment 122 having at least one bend.

In one embodiment, the linking segments are made of a first material and the winding segments are made of a second material different from the first material. For example, the winding segments may be made of a shape memory material, for example, nitinol and the second material may be made of stainless steel. Adjacent winding and linking segments may be fused one to the other, for example by soldering, or adhesively bonded one to the other or joined together via any of the other modalities discussed in this disclosure.

In another embodiment, the linking segments (or expandable segments) and the winding segments are made from the same material where the linking segments (or expandable segments) have been subjected to a different treatment than the winding segments. For example, the linking segments (or expandable segments) may have been differently annealed than the winding segments, differently heat treated or subject to a different chemical treatment. The implant may be made from a shape memory material where the shape memory of the linking segments (or expandable segments) has been destroyed by being subject to a different treatment than the winding segments. Heat treatment typical for superelastic material such as nitinol occurs in the range of 500 C. By heating nitinol based linking segments to temperatures substantially in excess of 500 C and just below the melting point of about 1300 C, the superelastic properties of the linking material will be destroyed. Such a treatment may be accomplished by first heat treating the entirety of the shape memory material to set the shape of the coil and then by selectively heat treating the linking members to destroy the superelastic properties of the linking members.

Desirably, the curvature of the ends of the linking segments will be substantially similar to the curvature of the ends of the winding segments to avoid an excess concentration of stress at junctions between the linking segments and the winding segments.

Where the coil segment comprises individual segments which are joined together, and the various segments are subject to different treatments, heat, chemical or otherwise, the shape of the individual segments may be set prior to, during or subsequent to joining the segments together.

Similarly, where the coil segment is formed from a continuous strand or strip of material, segments of which are subjected to different treatments, the shape of the coil segment may be established prior to, during or subsequent to the treatment of the coil segment material.

Desirably, in those embodiments of the invention where the coil segment includes expandable links or linking segments, the coil segment will be constructed to allow for up to a 100% additional radial expansion or more of the segment following initial expansion of the segment to the maximum diameter attainable by expansion of the curved segments. The extent of the additional expansion provided by the expandable links or linking segments will depend on the choice of materials and the design of the expandable links or linking segments. For example, where the expandable link or linking segment comprises a plurality of bends, the extent of the additional expansion provided by the expandable link or linking segment will depend on the total length of the expandable link or linking segment when it is unbent and on the extent to which the expandable link or linking segment unbends during expansion.

Any of the inventive stents disclosed herein may be constructed and arranged so that at least a portion of the stent tapers when the stent is in the expanded state. The stent may taper from one end to the other end or a portion of the stent may have a taper and the remainder of the stent is of constant diameter in the expanded state. The stent may include one or more portions of increasing diameter which are followed by one or more portions of decreasing diameter in the expanded state.

The inventive stents disclosed herein may be constructed of any size and be of any diameter suitable for use in a bodily vessel or other body structures. Desirably, the inventive stents will range in length from about 3 mm to about 100 mm or longer. Also desirably, the inventive stents will, in the expanded state, range in diameter from about 1.5 mm to about 25 mm or larger. The expandable links will desirably allow up to a doubling or more of the diameter of the stent beyond the maximum diameter attainable by expansion of the curved segments of the stent.

As discussed above, in any of the inventive medical devices (e.g. stents, grafts, vena cava filters, vaso-occlusive devices and other coil based medical devices) disclosed herein, at junctions where segments of different material are joined together, or junctions where adjacent segments are differently treated, the curvature of the adjacent ends of the adjacent segments will desirably be substantially similar to one another to avoid an excess concentration of stress at the junctions between the expandable links and the curved segments.

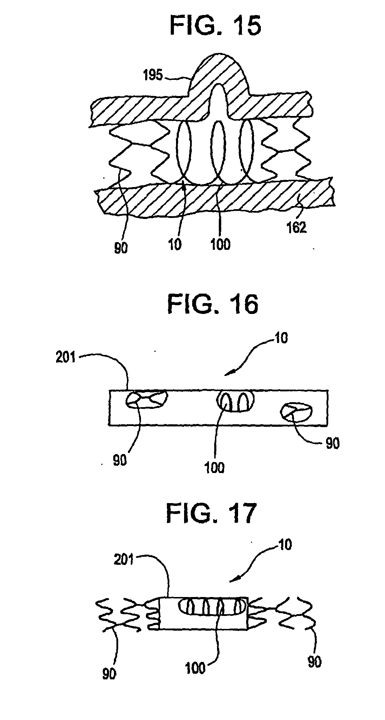

The invention is also directed to covered stents or grafts where the inventive stents disclosed herein serve as the framework as well as to lined stents. Any suitable covering, lining or graft materials may be used including collagen, polyethylene terephthalate (PET), polyethylene, polypropylene, polyamides, polytetrafluoroethylene (PTFE), expanded polytetrafluoroethylene and any other suitable polymeric material. Metal foils may also be disposed about the stent framework. The entirety of the stent may have a covering 201 as shown in Fig. 16 or a liner or the covering 201 or liner may be limited to one or more portions of the stent as shown in Fig. 17. In one embodiment of the invention, the cover or liner is provided only in the coil region(s) of the stent. Where more than one coil region is provided some or all of the coils may have a covering or liner. It is also within the scope of the invention for a portion, but not the entirety, of a coil to have a cover or liner. Where the inventive stents are to be positioned in a vessel in the region of an aneurysm, it may be desirable to include a covering or liner with the stent in the region of the stent that will be adjacent to the aneurysm.

It is noted, for the purposes of this disclosure, that the term "bend" does not refer to a specific method of construction. For example, the expandable links and more specifically the bent segments may be formed by laser cutting or chemically etching a curved pattern in a material. The expandable links may also be formed by physically bending a wire or other piece of material.

The inventive medical devices may include suitable radiopaque coatings. For example, the inventive medical devices may be coated with gold or other noble metals or sputtered with tantalum or other metals. The inventive medical devices may also be made directly from a radiopaque material to obviate the need for a radiopaque coating or may be made of a material having a radiopaque inner core. For example, the inventive medical devices may be made of nitinol disposed about a platinum core. Such a construction is disclosed in US 6,165,178. Any of the other coil materials and constructions disclosed in US 6,165,178 for coils may also be employed in the inventive medical devices disclosed herein. Other radiopaque metals which may be used include platinum, platinum-tungsten, palladium, platinum-iridium, rhodium, tantalum, or alloys or composites of these metals.

The inventive medical devices may also be provided with various biocompatible coatings to enhance various properties of the inventive medical devices. For example, the inventive medical devices may be provided with lubricious coatings or other polymeric coatings. An example of a suitable polymeric coating is PTFE.

The inventive stents may include one or more coatings which comprise one or more therapeutic agents, cellular materials, polymeric agents

The therapeutic agent may be non-genetic or genetic. Suitable non-genetic therapeutic agents include anti-thrombogenic agents such as heparin, heparin derivatives, urokinase, and PPack (dextrophenylalanine proline arginine chloromethyl ketone), anti-proliferative agents such as enoxaprin, angiopeptin, or monoclonal antibodies capable of blocking smooth muscle cell proliferation, hirudin, and acetylsalicylic acid, anti-inflammatory agents such as dexamethasone, prednisolone, corticosterone, budesonide, estrogen, sulfasalazine, and mesalamine, antineoplastic/antiproliferative/anti-miotic agents such as paclitaxel, 5-fluorouracil, cisplatin, vinblastine, vincristine, epothilones, endostatin, angiostatin and thymidine kinase inhibitors, anesthetic agents such as lidocaine, bupivacaine, and ropivacaine, anti-coagulants such as D-Phe-Pro-Arg chloromethyl keton, an RGD peptide-containing compound, heparin, antithrombin compounds, platelet receptor antagonists, anti-thrombin antibodies, anti-platelet receptor antibodies, aspirin, prostaglandin inhibitors, platelet inhibitors and tick antiplatelet peptides, vascular cell growth promoters such as growth factor inhibitors, growth factor receptor antagonists, transcriptional activators, and translational promoters, vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin, cholesterol-lowering agents; vasodilating agents; and agents which interfere with endogenous vascoactive mechanisms.

Suitable genetic materials include anti-sense DNA and RNA, DNA coding for anti-sense RNA, tRNA or rRNA to replace defective or deficient endogenous molecules, angiogenic factors including growth factors such as acidic and basic fibroblast growth factors, vascular endothelial growth factor, epidermal growth factor, transforming growth factor α and β, platelet-derived endothelial growth factor, platelet-derived growth factor, tumor necrosis factor α, hepatocyte growth factor and insulin like growth factor, cell cycle inhibitors including CD inhibitors, thymidine kinase ("TK") and other agents useful for interfering with cell proliferation, the family of bone morphogenic proteins ("BMP"s"), BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Any of BMP-2, BMP-3, BMP-4, BMP-5, BMP-6 and BMP-7 are particularly desirable. These dimeric proteins can be provided as homodimers, heterodimers, or combinations thereof, alone or together with other molecules. Alternatively or, in addition, molecules capable of inducing an upstream or downstream effect of a BMP can be provided. Such molecules include any of the "hedgehog" proteins, or the DNA"s encoding them.

Suitable cellular materials include cells of human origin (autologous or allogeneic) or from an animal source (xenogeneic), genetically engineered if desired to deliver proteins of interest at the transplant site. The delivery media can be formulated as needed to maintain cell function and viability.

Suitable polymer coating materials include polycarboxylic acids, cellulosic polymers, including cellulose acetate and cellulose nitrate, gelatin, polyvinylpyrrolidone, cross-linked polyvinylpyrrolidone, polyanhydrides including maleic anhydride polymers, polyamides, polyvinyl alcohols, copolymers of vinyl monomers such as EVA, polyvinyl ethers, polyvinyl aromatics, polyethylene oxides, glycosaminoglycans, polysaccharides, polyesters including polyethylene terephthalate, polyacrylamides, polyethers, polyether sulfone, polycarbonate, polyalkylenes including polypropylene, polyethylene and high molecular weight polyethylene, halogenated polyalkylenes including polytetrafluoroethylene, polyurethanes, polyorthoesters, proteins, polypeptides, silicones, siloxane polymers, polylactic acid, polyglycolic acid, polycaprolactone, polyhydroxybutyrate valerate and blends and copolymers thereof, coatings from polymer dispersions such as polyurethane dispersions (BAYHDROL^{®}, etc.), fibrin, collagen and derivatives thereof, polysaccharides such as celluloses, starches, dextrans, alginates and derivatives, hyaluronic acid, squalene emulsions. Desirably, polyacrylic acid, available as HYDROPLUS^{®} (Boston Scientific Corporation, Natick, Mass.), and described in U.S. Pat. No. 5,091,205, the disclosure of which is hereby incorporated herein by reference, may be used. Also desirably, the polymer may be a copolymer of polylactic acid and polycaprolactone. Other materials include selected medical-grade biodegradable materials such as PGA-TMC, Tyrosine-Derived Polycarbonates and arylates, polycaprolactone co butyl acrylate and other co polymers, Poly-L-lactic acid blends with DL-Lactic Acid, Poly(lactic acid-co-glycolic acid), polycaprolactone co PLA, polycaprolactone co butyl acrylate and other copolymers, Tyrosine-Derived Polycarbonates and arylate, poly amino acid, polyphosphazenes, polyiminocarbonates, polydimethyltrimethylcarbonates, biodegradable CA/PO₄'s, cyanoacrylate, 50/50 DLPLG, polydioxanone, polypropylene fumarate, or polydepsipeptides.

Other suitable coatings include macromolecules such as chitosan and Hydrozylpropylmethylcellulose. Surface erodible materials may also be used. Coatings may also comprise maleic anhydride copolymers, zinc-calcium phosphate and amorphous polyanhydrides.

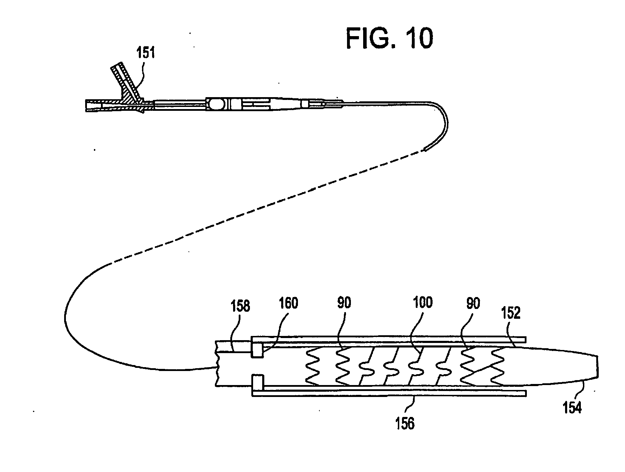

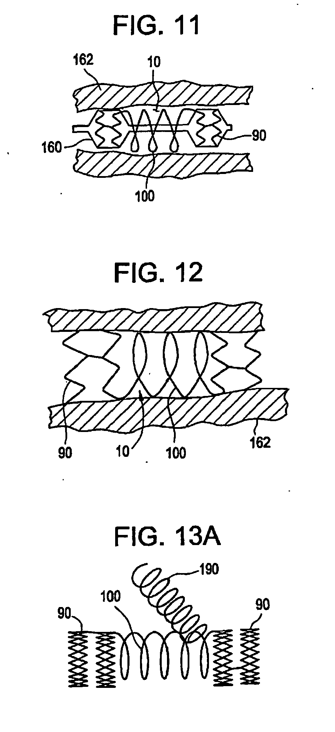

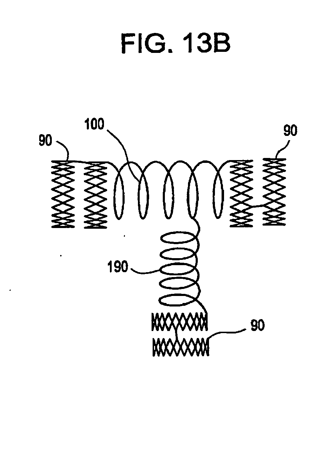

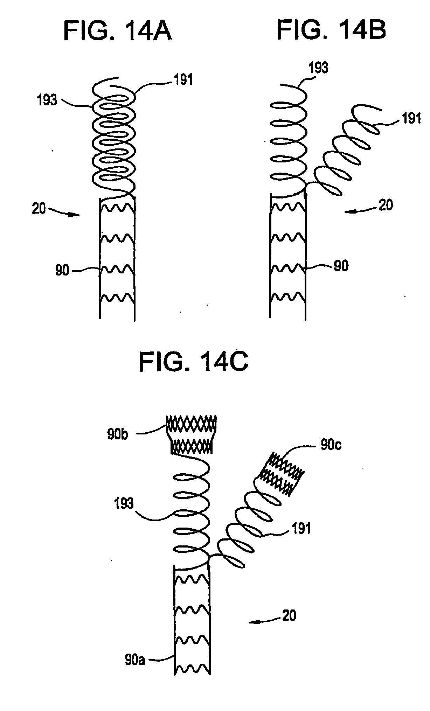

The inventive medical devices may also be provided with a sugar or more generally a carbohydrate and/or a gelatin to maintain the inventive medical devices on a balloon during delivery of the medical device to a desired bodily location. Other suitable compounds for treating the inventive medical devices include biodegradable polymers and polymers which are dissolvable in bodily fluids. Portions of the interior and/or exterior of the inventive medical devices may be coated or impregnated with the compound. Mechanical retention devices may also be used to maintain the inventive medical devices on the balloon during delivery.
The inventive medical devices may also be provided in whole or in part with one or more of the above therapeutic agents, polymeric coatings or the like. Where multiple therapeutic agents are provided, the different coatings may release the drugs at different rates. For example, one therapeutic agent may be released at a fast rate and another therapeutic agent may be released at a slow rate. Where multiple polymeric coatings are provided, the coatings may degrade or erode at different rates.
The invention is also directed to a medical implant comprising at least one and desirably two or more non-coil segments and one or more coil segments. The inventive implant may be made in the form of a stent as shown in the figures above, in the form of a vena cava filter or in the form of a vaso-occlusive device. To that end, any of the coil based vaso-occlusive devices disclosed in US 6,165,178 may be provided with one or more non-coil segments for anchoring the device and with coil segments as disclosed herein.
The inventive stents may advantageously be implanted by first expanding the non-coil segments or allowing the non-coil segments to expand and then expanding or contracting the coil segments to a desired length. As such, the invention can also be used in a method of implanting a stent having one or more coil portions and one or more non-coil portions. One or more of the non-coil portions is expanded or allowed to expand in order to anchor the stent in a desired region in a bodily vessel. Thereafter, the one or more coil portions are expanded or contracted to a desired length. Optionally, any remaining unexpanded non-coil portions may then be expanded or allowed to expand.
Where the stent has segments exhibiting self-expanding characteristics, the self expanding segments of the stent and the coil segment may be held in place on the catheter via a restraint such as a sheath. The sheath may then be retracted to allow the self-expanding segments to self-expand and to allow for deployment of the coil segment.
Where the coil segment includes expandable links as discussed above, an additional force may be applied to the stent via an expandable device such as a balloon in order to complete the deployment of the stent. The balloon may be used to apply a force to the stent and thereby expand the expandable link(s).
In accordance with the inventive method, a stent delivery catheter such as that shown generally at 150 in Fig. 10 is provided. Catheter 150 includes a manifold 151 at the proximal end and an inner tube 152 which terminates in a tip 154 at the distal end. Stent 10 is disposed about the distal end of inner tube 1 52. Stent 10 may be any of the inventive stents disclosed herein. Retractable sheath 156 covers stent 100. Pull collar 160 is attached to retractable sheath 156. Pull wire 158 extends from pull collar 160 to the proximal end of the catheter.
The distal end of catheter 100 is inserted in a bodily vessel and advanced to a desired location in the body. Retractable sheath 1 56 is retracted by pulling proximally on pull wire 158. Where stent 10 includes self-expanding segments, as retractable sheath 156 is retracted, the self-expanding segments 90 of stent 10 expand and the coil segment is deployed.
Where stent 10 includes balloon expandable segments 90 or expandable links within coil segment 100, catheter 1 50 may be withdrawn and, as shown in Fig. 11, a balloon catheter 160 advanced and positioned with stent 10. Stent 10 in Fig. 11 is not fully expanded. Balloon catheter160 is then inflated thereby expanding the expandable links and expandable segments 100 of the stent thereby seating the stent in the desired location in bodily vessel 162. The balloon catheter is then withdrawn. The seated stent is shown schematically in Fig. 12.
A stent delivery catheter which includes a balloon can be used so that the stent may be seated without the need to withdraw the stent delivery catheter and insert a balloon catheter. The catheter of Fig. 10 may be modified by including a balloon disposed between the stent and the inner tube and including an inflation lumen in fluid communication with the balloon.
Where the stent has multiple balloon expandable segments, for example, where the proximal and distal segments of the stent are balloon expandable, a delivery catheter having two or more separate balloons may be provided to inflate each balloon expandable segment of the stent. The invention also contemplates delivering and deploying such a stent using a catheter having two enlarged portions and a connecting portion of smaller cross-section. Such a balloon may be provided in the form of a dog-bone shape as shown at 160 in Fig. 11, thereby allowing for balloon inflation of the balloon expandable ends of the stent without inflation of the coil segment of the stent. Such a dog bone shaped balloon is considered inventive as is a catheter comprising a dog bone shaped medical balloon.
The inventive stents may also be delivered through a microcatheter and post inflated with a medical balloon. Microcatheters are described in US 5,540, 680, US 4,884, 579 and US 4, 739, 768.
The invention is also directed to a stent such as those shown schematically in Figs. 1 and 2 comprising a coil segment 104 and a tubular, non-coil segment108. In some embodiments, the non-coil coil segment will be balloon expandable. In other embodiments, the tubular, non-coil segment will be self- expandable. Typically, both the first end and the second end of the stent will be a tubular, non-coil segment as shown in Fig. 1a although embodiments in which only one end is a non-coil segment, as shown in Fig. 2, are within the scope of the invention. It is also within the scope of the invention to provide stents having a plurality of non-coil segments.
Typically, in the various embodiments of the invention, the segment which is in the form of a coil will be made of spring steel. Other suitable materials may also be used.
The inventive stents may also be provided in the form of bifurcated stents. As an example of one such inventive stent, a stent such as that shown at 10 in Fig.13a includes a sidebranch 190 which extends from coil segment 100. Each side of coil segment 100 has a non-coil segment 90 extending therefrom. The invention is also directed to bifurcated stents where the entirety of the sidebranch is a non-coil stent segment and to embodiments where a coil segment with a non-coil segment is present only in the sidebranch. Another embodiment is shown in Fig. 13b. In the embodiment of Fig.13b, sidebranch 190 includes an optional non-coil segment 90.
In another embodiment of a bifurcated stent, a bifurcated having two branches of unequal length may be provided. At least one of the trunk and the two branches is in the form of a coil stent. Desirably, one or both of the branches are in the form of a coil stent and the main branch of the stent is balloon expandable. Where one or more of the branches are in the form of coil stents, the coils may optionally further compriseballoon expandable portions. Where more than one coil stent is present, each of the coils may be wound in the same direction or, optionally, in opposing directions. The latter case of counter-wound coils may prove particularly beneficial in that it may allow for the mainbranch and sidebranch of a stent to be delivered together and then easily separated. A schematic illustration of a bifurcated stent having counterwound coils which form branch 191 and second branch 193 is shown generally at 20 in Fig. 14a prior to deployment and in Fig. 14b post deployment.

In many of the inventive bifurcated stents disclosed herein, the sidebranch stent may optionally be provided by pushing a second stent in between the coils of the mainbranch stent.

In any of the bifurcated stents disclosed herein, the sidebranch may be of the same diameter as the mainbranch of the stent or may be of different diameter than the mainbranch. For example, the sidebranch may be of smaller diameter than the mainbranch.

More generally, the invention is also directed to stents having two or branches extending therefrom where the stent has coil segments and non-coil segments.

The inventive stents may be manufactured via a variety of methods. In accordance with one method, the individual segments of the stent are provided and then secured to one another. Adjacent segments may be secured to one another via the use of adhesives or via welding. Welding of adjacent segments may prove particularly beneficial where the stent segments are made of metal.

The inventive stents may also be made from a single piece of material. For example, a sheet of super-elastic material may be provided and a stent pattern provided therein by laser cutting, etching, mechanical cutting whether robotic or otherwise or any other suitable method. The stent pattern will include a portion which is in the form of a coil and one or more portions which are not in the form of a coil but which have another non-coil stent pattern. The sheet of material may then be rolled to form a stent. Optionally, opposing edges of the non-coil portion of the stent may be welded to one another. The coil portion may then be straightened. Upon insertion of the stent in the body and expansion of the stent, the coil portion will assume its coil configuration.

The inventive stents may likewise be made from a tube. One or more portions of the tube are provided with a coil design, as by laser cutting etching, mechanical cutting and the like and one or more portions of the tube are provided with a non-coil pattern.

The invention is also directed toward the above methods of manufacturing a stent from a sheet or a tube.

The invention is also directed to methods of manufacturing any of the inventive stents disclosed herein. In accordance with one inventive embodiment, a coil segment is provided as is a non-coil segment. Any of the coil segments and non-coil segments disclosed herein may be used. The coil segment is attached to the non-coil segment through any suitable method include via welding or the use of adhesives. Optionally, additional non-coil segments may be attached at the other end of the coil segment. Moreover, additional coil segments may be attached to the non-coil segments.
The inventive stents may find use in the cerebral arteries as well as in the coronary arteries, the peripheral arteries and the arteries of the neck. The inventive stents may find used in the aorta or vena cava. The stents of the present invention are not limited to use in the vascular system and may also be advantageously employed in other body structures, including but not limited to arteries, veins, biliary ducts, urethras, fallopian tubes, bronchial tubes, the trachea, the esophagus, the prostate and the bowel. The inventive stents may be used interarterially in the brain, deployed across the neck of an aneurysm as well as in occlusions in bodily vessels. The size of the inventive stents will be appropriate for the intended usage of the stent. The inventive stents may be used to support other medical devices or may be used as filters.
In cases where the inventive stents are deployed across the neck of aneurysms, the coil segment of the inventive may serve as a flow impediment or an embolic material impediment. A schematic illustration showing an inventive stent with a coil segment extending across aneurysm 195 is shown in Fig. 15. In the case of an intercranial aneurysm which occurs at a point of bifurcation of healthy vessels and where it is desirable to block blood flow to the aneurysm but undesirable to block blood flow to or from healthy collateral vessels, an inventive bifurcated stent may prove useful. The coil segments of the inventive stents, because of their flexibility, may also reduce the likelihood of vessel straightening, which is undesirable intercranially.
Also, the coil portion of any of the inventive stents disclosed herein may be delivered to an aneurism and individual coils which are separate from the coil portion of the stent delivered to the aneurism sack. The coils may be disposed in the aneurism sack by being pushed out of the stent between adjacent turns of the coil and into the sack. The coils which are placed in the aneurism sack may be made of any suitable material including platinum.
Where the inventive stents are used in cerebral arteries, the coil segment desirably will have an outer diameter of no more than 6 mm when deployed. More desirably, the stent as a whole will have an outer diameter of no more than 5 mm. Also, when used in cerebral arteries, the inventive stents will desirably have a length of no more than 20 mm.
The above disclosure is intended to be illustrative and not exhaustive.

## Claims

1. A stent comprising a plurality of segments, including first and second end segments in a form other than a coil and being balloon-expandable or self-expandable, **characterized in that** at least one further segment (100) is provided in the form of a coil, and **in that** the first and second end segments are each formed of a plurality of braided filaments.

2. The stent of claim 1, comprising only one segment which is in the form of a coil and which connects the first and second end segments.

3. The stent of claim 2, wherein the first and second end segments are self-expandable.

4. The stent of claim 3, wherein the filaments are made of spring steel.

5. The stent of claim 2, wherein the first and second end segments are balloon-expandable.

6. The stent of claim 5, wherein the first and second end segments are each in the form of a tube comprising a plurality of interconnected serpentine segments.

7. The stent of claim 1, wherein the segment which is in the form of a coil is made of spring steel.

8. The stent of claim 1, comprising a tubular, non-coil segment.

9. The stent of claim 8, wherein the tubular, non-coil segment is balloon-expandable.

10. The stent of claim 8, wherein the tubular, non-coil segment is self-expandable.

11. The stent of claim 8, wherein the first and second end segments are tubular, non-coil segments.

12. The stent of claim 8, where the coil segment is made of spring steel.

13. The stent of claim 8, wherein the coil segment has an outer diameter of no more than 6 mm when deployed.

14. The stent of claim 13, having an outer diameter of no more than 6 mm when deployed.

15. The stent of claim 14, having a length of no more than 20 mm.

16. A method of manufacturing a stent according to one of the claims 1 to 15, comprising the steps of:
**•** providing a coil segment and a non-coil segment, first and second non-coil segments being formed of a plurality of braided filaments,
• attaching the coil segment to the non-coil segment.

17. The method of claim 16, wherein the coil segment is adhesively bonded to the non-coil segment.

18. The method of claim 16, wherein the coil segment is welded to the non-coil segment.

## Patentansprüche

1. Stent, umfassend eine Mehrzahl Segmente, umfassend erste und zweite Endsegmente in einer anderen Form als einer Spule, die ballonexpandierbar oder selbstexpandierbar sind, **dadurch gekennzeichnet, dass** mindestens ein weiteres Segment (100) in Form einer Spule vorgesehen ist, und **dadurch**, dass die ersten und zweiten Endsegmente jeweils aus einer Mehrzahl geflochtener Filamente bestehen.

2. Stent nach Anspruch 1, umfassend nur ein Segment, das in Form einer Spule vorliegt, und das die ersten und zweiten Endsegmente verbindet.

3. Stent nach Anspruch 2, wobei die ersten und zweiten Endsegmente selbstexpandierbar sind.

4. Stent nach Anspruch 3, wobei die Filamente aus Federstahl hergestellt sind.

5. Stent nach Anspruch 2, wobei die ersten und zweiten Endsegmente ballonexpandierbar sind.

6. Stent nach Anspruch 5, wobei die ersten und zweiten Endsegmente jeweils in Form eines Rohres vorliegen, das eine Mehrzahl miteinander verbundener gewundener Segmente umfasst.

7. Stent nach Anspruch 1, wobei das Segment, das in Form einer Spule vorliegt, aus Federstahl hergestellt ist.

8. Stent nach Anspruch 1, umfassend ein rohrförmiges Nicht-Spulen-Segment.

9. Stent nach Anspruch 8, wobei das rohrförmige Nicht-Spulen-Segment ballonexpandierbar ist.

10. Stent nach Anspruch 8, wobei das rohrförmige Nicht-Spulen-Segment selbstexpandierbar ist.

11. Stent nach Anspruch 8, wobei die ersten und zweiten Endsegmente rohrförmig Nicht-Spulen-Segmente sind.

12. Stent nach Anspruch 8, wobei das Spulen-Segment aus Federstahl hergestellt ist.

13. Stent nach Anspruch 8, wobei das Spulen-Segment einen Außendurchmesser von nicht mehr als 6 mm aufweist, wenn es entfaltet ist.

14. Stent nach Anspruch 13, der einen Außendurchmesser von nicht mehr als 6 mm aufweist, wenn er entfaltet ist.

15. Stent nach Anspruch 14, der eine Länge von nicht mehr als 20 mm aufweist.

16. Verfahren zum Herstellen eines Stents nach einem der Ansprüche 1 bis 15, umfassend die folgenden Schritte:
• Bereitstellen eines Spulen-Segments und eines Nicht-Spulen-Segments, wobei die ersten und zweiten Nicht-Spulen-Segmente aus einer Mehrzahl geflochtener Filamente bestehen,
• Anbringen des Spulen-Segments an dem Nicht-Spulen-Segment.

17. Verfahren nach Anspruch 16, wobei das Spulen-Segment haftend mit dem Nicht-Spulen-Segment verbunden ist.

18. Verfahren nach Anspruch 16, wobei das Spulen-Segment an das Nicht-Spulen-Segment geschweißt ist.

## Revendications

1. Stent comprenant une pluralité de segments, y compris des premier et second segments d'extrémité sous une forme autre qu'une spirale et qui peuvent subir une expansion par ballon ou automatique, **caractérisé en ce qu'**au moins un autre segment (100) est prévu sous la forme d'une spirale, et **en ce que** les premier et second segments d'extrémité sont chacun formés d'une pluralité de filaments tressés.

2. Stent selon la revendication 1, comprenant uniquement un segment qui est sous la forme d'une spirale et qui connecte les premier et second segments d'extrémité.

3. Stent selon la revendication 2, dans lequel les premier et second segments d'extrémité sont à expansion automatique.

4. Stent selon la revendication 3, dans lequel les filaments sont réalisés en acier à ressort.

5. Stent selon la revendication 2, dans lequel les premier et second segments d'extrémité sont à expansion par ballon.

6. Stent selon la revendication 5, dans lequel les premier et second segments d'extrémité sont chacun sous la forme d'un tube comprenant une pluralité de segments en serpentin interconnectés.

7. Stent selon la revendication 1, dans lequel le segment qui est sous la forme d'une spirale est réalisé en acier à ressort.

8. Stent selon la revendication 1, comprenant un segment tubulaire différent d'une spirale.

9. Stent selon la revendication 8, dans lequel le segment tubulaire différent d'une spirale est à expansion par ballon.

10. Stent selon la revendication 8, dans lequel le segment tubulaire différent d'une spirale est à expansion automatique.

11. Stent selon la revendication 8, dans lequel les premier et second segments d'extrémité sont des segments tubulaires différents d'une spirale.

12. Stent selon la revendication 8, dans lequel le segment en spirale est réalisé en acier à ressort.

13. Stent selon la revendication 8, dans lequel le segment en spirale possède un diamètre externe ne dépassant pas 6 mm lorsqu'il est déployé.

14. Stent selon la revendication 13, ayant un diamètre externe ne dépassant pas 6 mm lorsqu'il est déployé.

15. Stent selon la revendication 14, ayant une longueur ne dépassant pas 20 mm.

16. Procédé de fabrication d'un stent selon l'une quelconque des revendications 1 à 15, comprenant les étapes consistant à :
• fournir un segment en spirale et un segment différent d'une spirale, des premier et second segments différents d'une spirale étant formés d'une pluralité de filaments tressés,
• relier le segment en spirale au segment différent d'une spirale.

17. Procédé selon la revendication 16, dans lequel le segment en spirale est collé de manière adhésive au segment différent d'une spirale.

18. Procédé selon la revendication 16, dans lequel le segment en spirale est soudé au segment différent d'une spirale.
